# EUROPEAN PATENT SPECIFICATION

(11) **EP 2 143 445 B1**
(45) Date of publication and mention of the grant of the patent: **04.09.2013**
(21) Application number: 08751979.9
(22) Date of filing: 23.04.2008
(51) Int. Cl.: A61K 47/14, A61K 9/70, A61K 31/192, A61K 47/08, A61K 47/30, A61P 29/00

(54) **MEDICATED PATCH**
MIT ARZNEISTOFF VERSETZTES PFLASTER
TIMBRE MÉDICAMENTEUX

(30) Priority: 23.04.2007 JP 2007113200
(43) Date of publication of application: 13.01.2010
(73) Proprietor: Hisamitsu Pharmaceutical Co., Inc., Tosu-shi Saga 841-0017 (JP)
(72) Inventor: SUZUKI, Shigeo, Tosu-shi Saga 841-0017 (JP); KOZUMA, Miyuki, Tosu-shi Saga 841-0017 (JP); TSURUDA, Kiyomi, Tosu-shi Saga 841-0017 (JP)
(74) Representative: Thomson, Craig Richard
(86) International application number: PCT/JP2008/057841
(87) International publication number: WO 2008/133272

(56) References cited:
- WO-A1-2005/110482
- WO-A1-2006/090839
- WO-A1-2006/090839
- JP-A- 60 155 111
- JP-A- 61 260 028
- JP-A- 62 240 613
- JP-A- 62 240 614

## Description

### [Technical Field]

The present invention relates to a patch. More particularly, the invention relates to a patch comprising an anti-inflammatory agent, wherein the infrequent events of dermatitis, etc. caused by an excessive light irradiation can be sufficiently prevented.

### [Background Art]

Preparations comprising an anti-inflammatory agent are often used for the alleviation of muscular pain and lower-back pain, in a wide range of dosage forms including oral preparations, injections and external applications.
Among these, with external applications, an excessive light irradiation may induce dermatitis, etc., although infrequently, due to the effect of photodecomposition reaction of anti-inflammatory agents. Accordingly, in an attempt to suppress dermatitis, etc. caused by light, several methods have been disclosed, including a method to suppress photodecomposition reaction of an anti-inflammatory agent (Patent document 1), a method wherein an ultraviolet absorbent is present in the backing of a patch comprising an anti-inflammatory agent (Patent document 2), and a method wherein titanium oxide is used in an external application comprising an anti-inflammatory agent (Patent document 3). In addition, some other methods to improve such effects have been proposed in recent years, including a method wherein an ultraviolet absorbent with a high skin-migration property is used in a base material of an external application in order to increase the migration of the ultraviolet absorbent into the skin (Patent document 4), and a method wherein soybean lecithin is blended (Patent document 5).

The Patent document 1 describes hexyl laurate as a cream-base for creams and as an ointment-base for ointments; the Patent document 4 describes hexyl laurate as an ointment-base for ointments, as a gel-base for gels, and as a cream-base for creams; the Patent document 5 describes hexyl laurate as a solubilizer of an organic ultraviolet absorbent and as a plasticizer of an adhesive base of a patch. However, in all these cases, hexyl laurate is described only as one of the alternatives of generally-used fatty acid esters, and none of these documents discloses any specific formulation in which hexyl laurate is used.

Patent document 1: JP, A, 60-155111
Patent document 2: WO 01/68061
Patent document 3: JP, A, 09-169658
Patent document 4: WO 06/090833
Patent document 5: WO 06/090839

### [Summary of Invention]

### [Technical Problem]

The inventors have confrontednewproblems in their successive research, such as an insufficient migration of the ultraviolet absorbent to the skin in a conventional patch, and a reduced adhesiveness to skin as well as discoloration of an adhesive base in a patch containing soybean lecithin as a skin-migration promoter for an ultraviolet absorbent.

Thus, an object of the present invention is to provide a value-added and satisfactory patch comprising an anti-inflammatory agent, which can effectively prevent the onset of dermatitis, etc. that occurs very rarely due to an excessive irradiation with light, and which exhibits sufficient anti-inflammatory and analgesic effects and solves the above-mentioned problems.

### [Solution to Problem]

During extensive research to attain the object above, the inventors found that a patch comprising hexyl laurate is able to solve all the above problems; the inventors continued their research and finally accomplished the present invention.

Namely, the invention relates to a patch comprising an adhesive base and a backing laminated thereon, wherein the adhesive base comprises an anti-inflammatory agent selected from ketoprofen, tiaprofenic acid and tolmetin, the ultraviolet absorbent 4-tert-butyl-4'-methoxydibenzoylmethane, hexyl laurate and polyterpene resin.
In addition, the invention relates to the patch, wherein the content of the hexyl laurate is between 0.1 mass% and 10 mass%.

As disclosed in the Patent documents 4 and 5, the onset of dermatitis, etc. caused by an excessive irradiation with light can be sufficiently suppressed by promoting the migration of an ultraviolet absorbent to the skin; however, its mechanism has not yet been clarified. It is considered as follows: when an ultraviolet absorbent migrates to the skin together with a photo-sensitive anti-inflammatory agent, the ultraviolet absorbent blocks the anti-inflammatory agent from the light not only in the preparation, but also in the skin.

The mechanism by which hexyl laurate in the patch of the present invention can increase the migration of an ultraviolet absorbent to the skin has not yet been clarified; it is considered as follows: lipophilic hexyl laurate having an ester linkage may serve as a mediator between the extremely lipophilic ultraviolet absorbent and the water-containing corneum of the skin, thereby increase the distribution of the ultraviolet absorbent to the skin.

### [Advantageous Effects of Invention]

The patch of the invention remarkably suppresses the onset of dermatitis caused by excessive light irradiation to the anti-inflammatory agent, and is capable of exerting an anti-inflammatory and analgesic effects without causing problems such as the discoloration of the adhesive base over time or the reduction in adhesiveness. Thus, the present invention provides a satisfactory patch, which can be expected as an extremely safe medicament in various applications.

The adhesive base of the patch of the invention comprises an anti-inflammatory agent selected from ketoprofen, tiaprofenic acid, and tolmetin. Ketoprofen, tiaprofenic acid, and tolmetin have skeletons similar to benzophenone. Ketoprofen is more particularly preferable.
The content of anti-inflamatory agent is not particularly limited as long as it shows a sufficient drug effect, however, it is used in 0.1-10 mass%, preferably in 0.2-5mass%, and more preferably in 1-4 mass%.

The ultraviolet absorbent blended in the adhesive base of the inventive patch is 4-*tert*-butyl-4'-methoxydibenzoylmethane (hereinafter, BM-DBM).
Although the content of the ultraviolet absorbent is not particularly limited, it is used in 0.01-20 mass%, preferably in 1-10 mass%, and more preferably in 2-7 mass%.

Furthermore, the adhesive base of the patch the present invention comprises hexyl laurate as a skin-migration promoter to promote migration of an ultraviolet absorbent into the skin without decreasing the level of migration of an anti-inflammatory agent to the skin.

In the patch of the present invention, the migration of the ultraviolet absorbent is appropriately adjusted such that the onset of dermatitis, etc. is effectively prevented and sufficient anti-inflammatory analgesic effect can be exerted. Preferably, the of migration is adjusted to, based on the measurement by a method described by the skin-migration experiment using hairless mouse, 5.0-20 µg/15mmφ, more preferably 5.0-15 µg/15mmφ, furthermore preferably 6.0-15 µg/15mmφ, even more preferably 6.0-10 µg/15mmφ, and particularly preferably 8.0-10 µg/15mmφ.

The content of hexyl laurate is not particularly limited as long as it shows a predetermined drug effect; however, considering the physical properties of preparations, it is used in 0. 1-10 mass%, preferably in 1-7 mass%, and more preferably in 2-5 mass%.

When the migration of an ultraviolet absorbent to the skin is not sufficient, dermatitis, etc. caused by excessive light irradiation cannot be effectively prevented. The patch of the present invention comprises hexyl laurate, thereby improves skin-migration of the ultraviolet absorbent while causes little or no change in the transdermal absorption of ketoprofen. Therefore, the patch of the invention can sufficiently exert generally-desired anti-inflammatory effect.

The ratio of the content of the anti-inflammatory agent, the ultraviolet absorber and hexyl laurate in the adhesive base of the patch of the invention is not particularly limited as long as the patch can exhibit the effect of the invention in such ratio. However, in order to prevent photodecomposition reaction of the anti-inflammatory agent and obtain sufficient effects of the invention, prevent crystal precipitation of the ultraviolet absorbent, and obtain desirable physical properties of the preparation including sufficient adhesive force, their ratio is (2):(1-10):(0.1-5), preferably (2):(2-5):(1-3), and more preferably (2):(2.2-3.8):(1.2-2.8).

In addition, since the ultraviolet absorbent such as BM-DBM is in a crystallized state at room temperature, a solubilizer may be simultaneously used in order to blend the ultraviolet absorbent in the adhesive base of the patch. As such solubilizer, normally-used solubilizers can be appropriately selected, such as, for example, crotamiton, diethyl sebacate, diisopropyl sebacate, diisopropyl adipate, soy lecitin, propylene glycol monocaprylate, propylene glycol dicaprylate.
Although the content of the solubilizer is not particularly limited, it is used in 0.1-10 mass%, preferably in 1-5 mass%, considering physical properties of the preparation.

The adhesive base of the inventive patch is not particularly limited as long as it is an adhesive base conventionally used in a patch; however, a rubber type, an acrylic type, a silicon type, or a mixture of two or more of these can be used.
The rubber base is not particularly limited, and is selected from a polyisoprene rubber such as a synthetic rubber or a natural rubber, a styrene-butadiene copolymer, astyrene-isoprene copolymer, a styrene-isoprene-styrene block copolymer, a styrene-butadiene-styrene block copolymer, polyisobutylene and the like.

Although the acrylic adhesive base is not particularly limited, a copolymer of alkyl (meth) acrylate, which is obtained from a C₄-C₁₈ aliphatic alcohol and (meth) acrylic acid, and vinylpyrrolidone or other functional monomers is suitable.

The acrylate polymer is not particularly limited; however, it is preferably a copolymer in which alkyl (meth)acrylates are copolymerized as a main component. The alkyl (meth)acrylate includes the alkyl (meth)acrylate in which, specifically, alkyl groups are a straight chain alkyl group or a branched alkyl group having 4 or more carbon atoms, such as butyl, pentyl, hexyl, heptyl, octyl, nonyl, decyl, undecyl, dodecyl, tridecyl; used either alone or in combination of two or more.

As a monomer which can be copolymerized with said alkyl (meth)acrylate, examples include monomers having a carboxyl group such as (meth) acrylic acid, itaconic acid, maleic acid and maleic anhydride; monomers having a sulfonate group such as styrene sulfonate, allyl sulfonate, sulfopropyl (meth)acrylate, (meth)acryloyloxynaphthalene sulfonate and acrylamido-methyl-propane sulfonate; monomers having a hydoxyl group such as hydroxyethyl (meth)acrylate and hydoxylpropyl (meth) acrylate; (meth) acrylic acid derivatives having an amido group such as (meth)acrylamide, dimethyl(meth)acrylamide, N-butyl(meth)acrylamide and N-methylol(meth)acrylamide; aminoalkyl (meth)acrylates such as aminoethyl (meth)acrylate, dimethylaminoethyl (meth)acrylate and butylaminoethyl (meth)acrylate; alkoxyalkyl (meth)acrylates such as methoxyethyl (meth) acrylate, ethoxyethyl (meth) acrylate and tetrahydrofurfuryl (meth)acrylate; alkoxyalkylene glycol (meth)acrylate such as methoxyethylene glycol (meth)acrylate, methoxydiethylene glycol (meth)acrylate, methoxypolyethylene glycol (meth)acrylate and methoxypolypropylene glycol (meth)acrylate; compounds having a vinyl group such as (meth)acrylonitrile, vinyl acetate, vinyl propionate, N-vinyl-2-pyrrolidone, methylvinyl pyrrolidone, vinylpyridine, vinylpiperidone, vinylpyrimidine, vinylpiperazine, vinylpyrazine, vinylpyrrole, vinylimidazole, vinylcaprolactam, vinyloxazole and vinylmorpholine, used either alone or in combination of two or more. The amount of the copolymerization of these monomers is appropriately set according to the weight-average molecular weight of the copolymer to be obtained.

As an adhesive base material of silicon type, polydimethylsiloxane is preferable.
The content of these polymers used in the adhesive base is, based on the weight of the total composition, 5-60 mass%, preferably 20-55 mass%, more preferably 25-50 mass%, considering the formation of adhesive layers and sufficient penetration.

In addition, the adhesive base may further appropriately comprise a tackifier, a plasticizer, a filler, an absorption promoter and the like.

A tackifier preferably has a softening point at 60°C-150°C; for example, rosin esters, hydrogenated rosin esters, rosin modified maleic acid esters, polyterpene resins and petroleum resins can be used. In particular, polyterpene resins are preferable because they promote the migration of the ultraviolet absorbent to the skin without reducing the migration of the anti-inflammatory agent to the skin.
The content of the tackifier is not particularly limited; however, considering the physical properties of a preparation, it is blended in 0.1-30 mass%, more preferably in 3-20 mass%, and even more preferably in 5-15 mass%.

Plasticizers include petroleum oils (*e.g*., paraffin type process oil, naphthene type process oil, aromatic type process oil, etc.), squalane, squalene, vegetable oils (*e.g*., olive oil, camellia oil, castor oil, tall oil, peanut oil), silicone oil, dibasic acid esters (*e.g.,* dibutylphthalate, dioctylphthalate, etc.), liquefied rubbers (*e.g*., polybutene, liquefied isoprene rubber), liquefied fatty acid esters (*e.g*., isopropyl myristate, diethyl sebacate, diisopropyl sebacate), diethylene glycol, polyethylene glycol, glycol salicylate, propylene glycol, dipropylene glycol, triacetin, trietyl citrate, crotamiton, and the like. In particular, liquid paraffin, liquefied polybutene, isopropyl myristate, diethyl sebacate and the like can be used.

As fillers, calcium carbonate, magnesium carbonate, silicates (*e.g.,* aluminum silicate, magnesium silicate, etc.), silicic acid, barium sulfate, calcium sulfate, calcium zincate, zinc oxide, titanium oxide, synthetic aluminum silicate, metal salt of fatty acid (*e.g*., zinc stearate, calcium stearate, etc.) and the like can be used.

The external application of the invention can be produced by any production methods known to those skilled in the art. In the following, production of a patch is described as an example.
The drug-containing adhesive layer can be produced by any method. For example, a drug-containing base composition is heat-melted, then spread onto an exfoliate paper or a backing, which is then affixed to a backing or a exfoliate paper to give the preparation. As an alternative production method, base ingredients including the drug are dissolved in a solvent such as toluene, hexane, or ethyl acetate, spread onto an exfoliate paper or a backing, dried to remove the solvent, then affixed to a backing or a exfoliate paper to give the patch.

As for the backing of the patch of the invention, a stretch or non-stretch backing may be used. For example, it is selected from an woven fabric, a knitted fabric, a non-woven fabric, polyurethane, polyester, polyvinyl acetate, polyvinylidene chloride, polyethylene, polyethylene terephthalate, an aluminum sheet, or a composite material thereof.
In addition, a liner is not particularly limited as long as it can protect the adhesive layer till when the medicate patch is applied to the skin; however, specifically, a liner includes a film of polyesters (e.g., polyethylene terephthalate), polyvinyl chloride and polyvinylidene chloride and the like; a laminated film of high-quality paper and polyolefin and the like. In such liners, the side that is in contact with the adhesive layer is preferably siliconized, so that the operation upon peeling off the liner from the preparation is facilitated.

### [Example]

The invention is explained in more detail by the experimental examples and examples below. The invention, however, is not limited to these examples. Here, in the examples, "%" always means mass%.

### [Experimental example 1]

### (Discoloration test of a soybean-lecithin-containing patch)

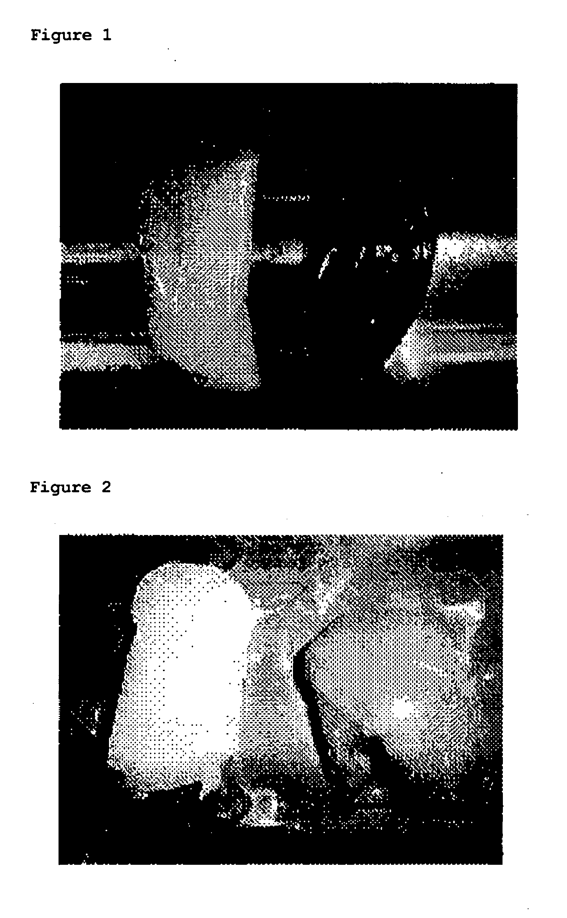

A homogenous adhesive base material was prepared using styrene-isoprene-styrene block copolymer (SIS), polyterpene resin, ketoprofen, and liquid paraffin as ingredients of the adhesive base, and stored at 140°C; during the storage, changes in colors was examined in the presence or absence of soybean lecithin in the above adhesive base.
Table 1 shows the specific content of each ingredient in Examinations 1 and 2, and Figs. 1 and 2 are the photographs showing the results.

**[Table 1]**

| Ingredient (mass%) | Examination 1 | Examination 2 |
|---|---|---|
| SIS | 30 | 30 |
| Polyterpene resin | 14 | 14 |
| BM-DBM | 3 | 3 |
| Soybean lecithin | 1 | - |
| Liquid paraffin | 45.5 | 46.5 |
| Ketoprofen | 2 | 2 |
| Others | 4.5 | 4.5 |

As is clear from the results shown in Table 1, Figs. 1 and 2 that the adhesive base comprising soybean-lecithin material showed a significant coloration due to heating.

### [Experimental example 2]

### (Adhesiveness test)

SIS copolymer, hydrogenated rosin glycerin ester and liquid paraffin were stirred with heating to give a solution. Then, ketoprofen, BM-DBM, a solubilizer, and soybean lecithin were added to the solution, and mixed to give a uniform solution of a base material for a plaster preparation. The base material was spread over a siliconized polyester film at a weight of 1 g per 70 cm², then was covered with a polyester woven fabric, transferred by pressure-bonding, and cut into a desired size to give a plaster preparation of the invention. The specific content of each ingredient of the patch in Examinations 3, 4 and 5, as well as results of the observation of probe tack and 180° peeling are shown in Table 2.

### <Probe tack test>

A tester described in ASTM D 2979 was used.
Material of the probe tip: Bakelite resin
Diameter of the bonding plane: 5 mm
Detaching rate: 5 mm/sec
Bonding load: 100 gf/cm²
Bonding duration: 1 sec

### <180° peeling test>

A patch sample with 20 mm width was adhered to a phenol resin, then immediately pressure-bonded by a reciprocation of a rubber roller having a mass of 850 g, and one edge of the patch is folded back to 180° and peeled off with a rate of 300 mm/min.

**[Table 2]**

| Ingredient (mass%) | Examination 3 | Examination 4 | Examination 5 |
|---|---|---|---|
| SIS | 34 | 34 | 28 |
| Hydrogenated rosin glycerin ester | 11 | 11 | 14 |
| BM-DBM | 3 | 3 | - |
| Soybean lecithin | 1 | 2 | - |
| Solubilizer | 2 | 2 | - |
| Liquid paraffin | 40.5 | 39.5 | 50 |
| Ketoprofen | 2 | 2 | 2 |
| Others | 6.5 | 6.5 | 6 |
| Probe tack (gf) | 49 | 42 | 60 |
| 180° peeling (gf) | 44 | 25 | 90 |

As is clear from the results shown in Table 2 that the soybean-lecithin-containing patches of Examinations 3 and 4 showed apparent reduction in adhesiveness.

### [Experimental example 3]

### (Solubility test of BM-DBM)

1 g of BM-DBM was mixed with 2 g of an additive, dissolved at 140°C, then the mixture was cooled to room temperature for 30 min and its state was observed. Table 3 shows the results; the symbol O indicates that no crystal precipitation of BM-DBM was observed even at 30 min after, and symbol X indicates that crystal precipitation was observed.

**[Table 3]**

| Additive | Solubility of BM-DBM | Additive | Solubility of BM-DBM |
|---|---|---|---|
| Olive oil | X | Starch acrylate | X |
| Oleyl oleate | X | L-menthyl glyceryl ether | O |
| Octyldodecyl myristate | X | Isostearic acid | X |
| Sorbitan trioleate | X | Isopropyl palmitate | X |
| Cetyl palmitate | X | Hexyl laurate | O |
| Myristyl myristate | X | Cetyl myristate | X |
| Silicon | X | Etyl linoleate | X |
| Diisopropyl sebacate | O | Sodium edetate | X |
| Stearic acid | X | Isopropylmyristate | X |
| Cator oil | X | Diisopropyladipate | X |

As is clear from the results shown in Table 3 that particularly good solubility of BM-DBM was observed for diisopropyl sebacate, L-menthyl glyceryl ether and hexyl laurate.

### [Examples 1 and 2]

### (Skin-migration test of BM-DBM and ketoprofen using hairless mouse)

The skin taken from a hairless mouse was divided into 4 equal parts and spread onto a filter paper (7 x 7 cm²) impregnated with physiological saline; then a preparation cut in a circle of 15 mm diameter (φ 15 mm) was adhered onto said skin. Upon this application, physiological saline was appropriately added to the filter paper not to dry the skin. Here, the test was performed on a plate placed in a water bath to maintain the temperature of the skin at 35°C. Six hours after the application, the skin of the hairless mouse was cut out in a circle of 20 mm diameter (φ 20 mm) in which the preparation was positioned at the center. The preparation was slowly peeled off from the cut skin, and BM-DBM and ketoprofen contained in the skin were extracted, as follows.
The skin was frozen stored, and then cut into thin sections on a petri dish using scissors while keeping the skin with tweezers, then the sections were placed in a 10-mL test tube. The petri dish, the scissors and the tweezers used for cutting were washed with 2 mL of methanol (twice with 1 mL each), and each washing liquid was collected in the test tube. Then, accurately 2 mL of acetonitrile solution an internal standard (benzophenone) in concentration of 150 µg/ml was added, the mixture was homogenized with a Polytron homogenizer for 3 min, then centrifuged at 3000 rpm for 10 min. The supernatant was filtered with a membrane filter, to give a sample solution.
After the extraction, by means of internal standard method using liquid chromatography, contents of BM-DBM and ketoprofen in the sample solution were quantified. Measurement conditions of the liquid chromatography were as follows.
Measurement device: Shimadzu LC-10A
Column used: TSK gel ODS-80TS (4.6I.D. x150 mm)
Eluent: 0.2% acetic acid solution: acetonitrile = 15:85
Flow rate of eluent: 1.2 ml/min
Column temperature: 40°C
Detector: UV 358 nm
The contents quantified were expressed in "µg/15mmφ" as migration of BM-DBM and ketoprofen from the preparation cut into 15-mm-diameter circle (area: 177 mm²) .

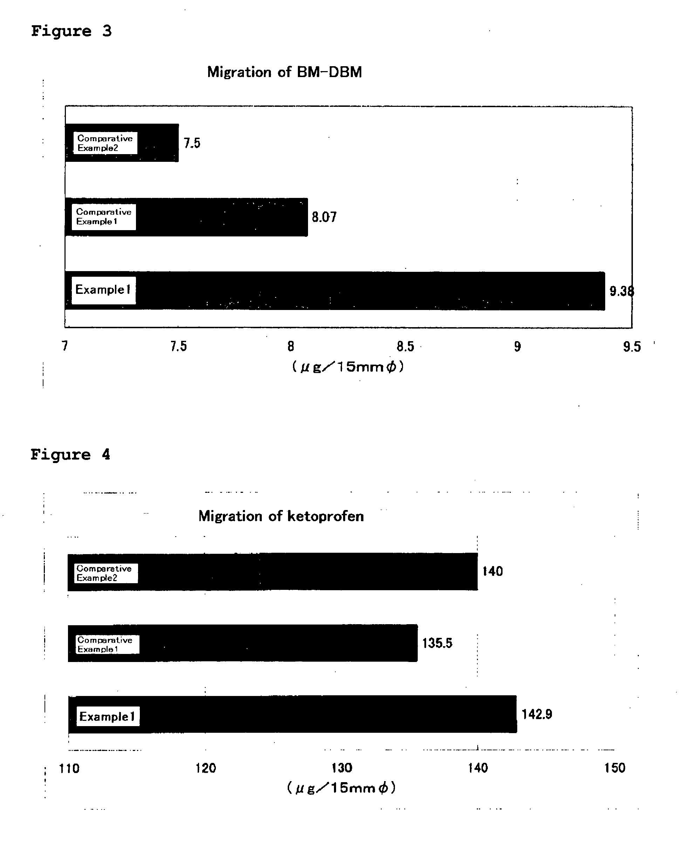

The preparations adhered were produced as follows. SIS copolymer, resin, and liquidparaffin were stirred to give a solution. Then, ketoprofen, BM-DBM, a solubilizer, and either diisopropyl sebacate or hexyl laurate were added to the solution, and mixed to give a uniform solution of the base material for a plaster preparation. The base material was spread over a siliconized polyester film at a weight of 1 g per 70 cm², then was covered with a polyester woven fabric, transferred by pressure-bonding, and cut into a desired size to give a plaster preparation of the invention. The specific content of each ingredient was shown in Table 4, and the measurement results of the migration of BM-DBM and ketoprofen were shown in Table 4, Figs. 3 and 4.

**[Table 4]**

| Ingredient (mass%) | Comparative example 2 | Comparative example 1 | Example 1 |
|---|---|---|---|
| SIS | 28.5 | 29 | 29 |
| hydrogenated rosin glycerin ester | 11.5 | 11 | 11 |
| BM-DBM | 3 | 3 | 3 |
| Hexyl laurate | - | - | 2 |
| Diisopropyl sebacate | - | 2 | - |
| Ketoprofen | 2 | 2 | 2 |
| Liquid paraffin | 55 | 53 | 53 |
| Migration of BM-DBM (µg/15mmφ) | 7.5 | 8.07 | 9.38 |
| Migration of ketoprofen (µg/15mmφ) | 140.0 | 135.5 | 142.9 |

As clearly shown from the results in Table 4, Figs. 3 and 4, it was confirmed that the patch of Example 1 comprising hexyl laurate increased the migration of BM-DBM without changing the migration of ketoprofen, compared to Comparative example 1 wherein diisopropyl sebacate was blended.

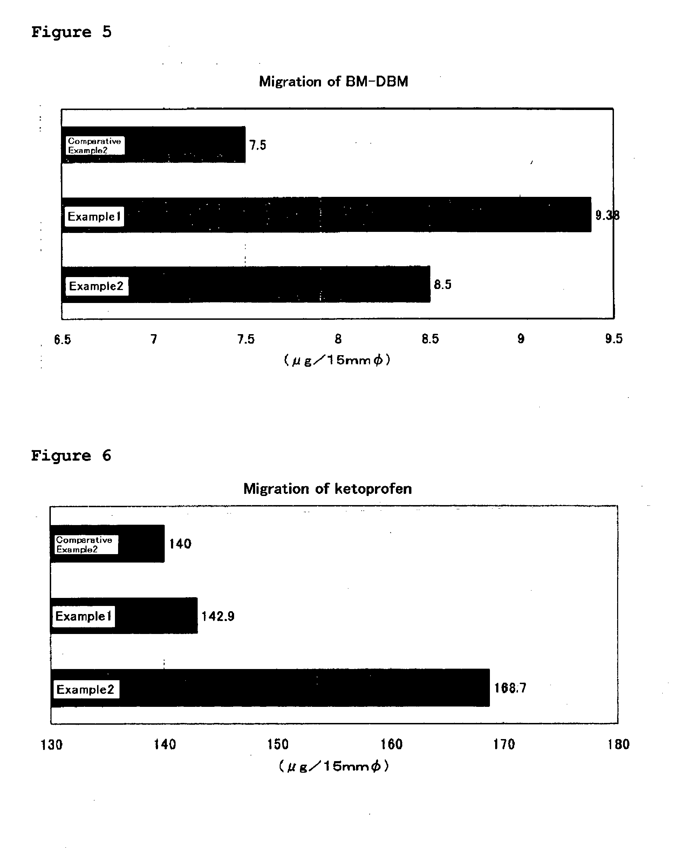

Next, a patch comprising polyterpene resin in addition to hexyl laurate, and a patch comprising neither hexyl laurate nor polyterpene resin were prepared in similar way. Table 5 shows the specific content of each ingredient, and Table 5, Figs. 5 and 6 show the measurement results of the migration of BM-DBM and ketoprofen. Example 1 in Table 5 is not according to the scope of the claims.

**[Table 5]**

| Ingredient (mass%) | Comparative example 2 | Example 1 | Example 2 |
|---|---|---|---|
| SIS | 28.5 | 29 | 28.5 |
| hydrogenated rosin glycerin ester | 11.5 | 11 | 6.5 |
| Polyterpene resin | - | - | 5 |
| BM-DBM | 3 | 3 | 3 |
| Hexyl laurate | - | 2 | 5 |
| Ketoprofen | 2 | 2 | 2 |
| Liquid paraffin | 55 | 53 | 50 |
| Observation of crystal at room temperature for 2 months | O | - | O |
| Migration of BM-DBM (µg/15mmφ) | 7.5 | 9.38 | 8.5 |
| Migration of ketoprofen (µg/15mmφ) | 140.0 | 142.9 | 168.7 |

As clearly shown from the results in Table 5, Figs. 5 and 6, it was confirmed that the patch of Example 2 using polyterpene resin in addition to hexyl laurate promoted the migration of BM-DBM as well as the migration of ketoprofen.
In contrast, the patch of Comparative example 2 comprising neither hexyl laurate nor polyterpene resin showed the smallest migration of BM-DBM and ketoprofen.
Meanwhile, neither of the patches of Example 2 nor Comparative example 2 showed any crystal precipitation during 2-month observation under room temperature.
Thus, it has been suggested that by blending poloyterpene resin into the adhesive base comprising hexyl laurate, a more effective patch can be obtained in which migration of BM-DBM and ketoprofen are appropriately adjusted.

### [Brief Description of Drawings]

Figure 1 shows a photograph comparing color changes of adhesive base comprising 1% soybean lecithin, before and after heating (left: immediately after production, right: at 140°C, after 4 hours).
Figure 2 shows a photograph comparing color changes of adhesive base without soybean lecithin, before and after heating (left: immediately after production, right: at 140°C, after 4 hours).
Figure 3 shows a graph comparing migration of BM-DBM by the application of the patches of Example 1, Comparative examples 1 and 2.
Figure 4 shows a graph comparing migration of ketoprofen by the application of the patches of Example 1, Comparative examples 1 and 2.
Figure 5 shows a graph comparing migration of BM-DBM by the application of the patches of Examples 1 and 2, and Comparative example 2.
Figure 6 shows a graph comparing migration of ketoprofen by the application of the patches of Examples 1 and 2, and Comparative example 2.

## Claims

1. A patch comprising an adhesive base and a backing laminated thereon, wherein the adhesive base comprises an anti-inflammatory agent, selected from ketoprofen, tiaprofenic acid, and tolmetin, the ultraviolet absorbent 4-tert-butyl-4'-methoxydibenzoylmethane; hexyl laurate, and polyterpene resin.

2. The patch according to Claim 1, wherein the content of hexyl laurate is between 0.1 mass% and 10 mass%.

3. The patch according to Claim 1 or 2, wherein the anti-inflammatory agent is ketoprofen.

## Patentansprüche

1. Ein Pflaster, das eine adhäsive Basis und einen darauf angebrachten Träger beinhaltet, wobei die adhäsive Basis ein entzündungshemmendes Mittel beinhaltet, das aus Ketoprofen, Tiaprofensäure und Tolmetin, dem Ultraviolett-Absorptionsmittel 4-Tert-butyl-4'-methoxydibenzoylmethan; Hexyllaurat und Polyterpenharz ausgewählt ist.

2. Pflaster gemäß Anspruch 1, wobei der Gehalt an Hexyllaurat zwischen 0,1 Masse% und 10 Masse% liegt.

3. Pflaster gemäß Anspruch 1 oder 2, wobei das entzündungshemmende Mittel Ketoprofen ist.

## Revendications

1. Un timbre comprenant une base adhésive et un support laminé sur celle-ci, dans lequel la base adhésive comprend un agent anti-inflammatoire, sélectionné parmi le kétoprofène, l'acide tiaprofénique et la tolmétine, l'absorbeur d'ultraviolet 4-tert-butyl-4'-méthoxydibenzoylméthane ; du laurate d'hexyle et de la résine polyterpène.

2. Le timbre selon la revendication 1, dans lequel la teneur en laurate d'hexyle est comprise entre 0,1 % en masse et 10 % en masse.

3. Le timbre selon la revendication 1 ou la revendication 2, dans lequel l'agent antiinflammatoire est le kétoprofène.
